# EUROPEAN PATENT APPLICATION

(11) **EP 2 014 294 A1**
(43) Date of publication of application: **14.01.2009**
(21) Application number: 07290883.3
(22) Date of filing: 13.07.2007
(51) Int. Cl.: A61K 35/28, G01N 33/569, C12N 5/00

(54) **Use of CD200 as a mesenchymal stem cells marker**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Vialle-Presles, Marie José

(57) **Abstract**

The invention relates to methods for phenotyping or isolating mesenchymal stem cells, wherein said method comprises detecting the expression of the marker CD200 at the surface of said cells

## Description

The present invention relates to the detection and/or isolation of mesenchymal stem cells (MSCs).

The term "mesenchymal stem cells" is commonly applied to adult tissue multipotential stem cells that give rise to cells of the skeletal connective tissues : osteoblasts (O), chondrocytes (C), tenocytes, bone marrow adipocytes (A) and hematopoiesis-supportive stromal cells that differentiate following a vascular smooth muscle (VSM) pathway. Certain observations suggest that they can also give rise to skeletal muscle (MS) cells and cardiac muscle (MC) cells (both sarcomeric muscle cells) and to endothelial cells (E). Some reports also indicate that they may give rise to non-mesodermic derivatives such as hepatocytes (endoderm) and neurons or astrocytes (neuro-ectoderm).

Mesenchymal stem cells can be isolated from several tissues, but bone marrow (BM) is the most common source.

Most information concerning MSCs is derived from *in vitro* studies of culture expanded cells.

Cultured bone marrow MSCs appear to be homogeneous according to a number of criteria. They are currently identified through a combination of physical, morphologic, phenotypic, and functional properties. They are defined as populations of adherent cells (CFU-Fs: colony-forming-unit-fibroblasts), able to give rise, when cultured in appropriate conditions, to adipocytes (A), osteoblasts (O) and chondrocytes (C) (PITTENGER et al., Science, 284, 143-7, 1999). Their morphology is usually that of elongated cells with, in particular in murine cultures, thin filopodial extensions. Flow cytometry studies in many laboratories have helped define a minimal consensus phenotype: cells being negative for CD45, CD34, CD31 and CD14, and positive for CD44, CD73, CD90 and CD105. However, the phenotypic properties of populations of cultured MSCs, and their homogeneity appear to depend, at least in part, on culture conditions.

Further, studies on native MSCs (i.e MSCs freshly isolated) have revealed great heterogeneity in terms of proliferative capacity and differentiation potential.

Although many questions remain concerning the developmental origin and differentiation mechanisms of mesenchymal stem cells, there is a consensus concerning their potential utility for therapeutic applications, as therapeutic agents in themselves, or as vehicles for the delivery of therapeutic genes (for review cf. BAKSH et al., J Cell Mol Med, 8, 301-16, 2004; DELORME et al., Regen Med, 1, 497-509, 2006; JAVAZON et al., Experimental Hematology, 32, 414-25, 2004).

The extensive proliferative potential of MSCs and their ability to differentiate into a variety of cell types make them an ideal candidate cell source for clinical tissue repair and regeneration. It has been shown that MSCs expanded *ex vivo* and administered to a damaged tissue such as bone, cartilage, tendon, ligament and myocardium were able to differentiate into cells of said tissue, allowing its regeneration. This regenerative ability appears not to be limited to cells of mesenchymal lineage, since it has also been reported for neurons and epithelial cells in skin, lung, intestine, and kidney. They also allow to enhance engraftment of hematopoietic stem cells after hematopoietic stem cell transplantation. It has also been observed in animal models that MSCs infused systematically have the ability to migrate to damaged tissues and organs and to engraft into it. In addition, it has been shown they have immunomodulatory properties (LE BLANC & RINGDEN, Curr Opin Immunol, 18, 586-91, 2006).

Further, MSCs can easily be modified genetically with various vectors (usually viral vectors) carrying therapeutic genes, and can therefore be useful for delivering said genes to target organs or tissues.

Therefore MSCs represent a privileged source of cells for a broad range of cell-based therapy strategies.

However, for cell therapy, it is essential to transplant well-defined and homogenous cell populations, in order to ensure reproducible results. Since it appears that the properties of MSCs populations may vary, depending *inter alia* on the method of isolation and on the culture conditions, there is a need to develop suitable markers allowing a better accuracy in the identification of MSCs, as well as a better characterisation of MSCs populations.

Also, it appears that MSCs undergo during culture various modifications, which are likely to affect their properties. This can be favourable or disadvantageous, depending on the therapeutic application contemplated. For instance, it has been reported that *in vitro* propagation of BM-derived MSCs decreases their homing to BM and spleen (ROMBOUTS & PLOEMACHER, Leukemia, 17, 160-70, 2003). Thus the use of native MSCs would be preferable for systemic administration.

However, the isolation of MSCs from primary tissues is hampered by the few number of sufficiently selective markers that are available.

The inventors have now succeeded in identifying a novel marker allowing to differentiate mesenchymal stem cells from hematopoietic cells in bone marrow, and also from other skeletal, but non BM-derived, cultured mesenchymal cells, i.e. periosteal cells (POCs) and synovial fibroblasts (SFbs).

This marker is the surface antigen CD200 (OX2). In humans, CD200 is expressed at the cell surface of thymocytes, B cells, T cells, neurons, kidney glomeruli, tonsil follicles, the syncytiotrophoblast and endothelial cells (WRIGHT et al., Immunology, 102, 173-79, 2001). It has immunoregulatory properties, delivering an inhibitory signal for the macrophage lineage (HOEK et al., Science, 290, 1768-71, 2000; YU et al., Transplantation, 80, 394-401, 2005). However, CD200 is not expressed on the haematopoietic cells of the bone marrow (MOREAUX et al., Blood, 108, 4194-97, 2006), and before now, it had not been detected at the surface of MSCs.

The present invention thus relates to a method for phenotyping a population of MSCs, preferably human MSCs, by detection of cell surface markers in this population wherein said method comprises detecting the expression of the surface marker CD200 in this population. The invention also provides a method for numbering MSCs in a biological sample, wherein said method comprises detecting the expression of the surface marker CD200 on said MSCs.

The present invention also relates to a method for isolating native mesenchymal stem cells, preferably human MSCs, wherein said method comprises providing a tissue sample containing mesenchymal stem cells, detecting the expression of CD200 in cells from this tissue sample, and isolating the CD200⁺ cells.

According to a preferred embodiment of the invention, said tissue sample is a bone marrow sample. However the method of the invention may also be used to isolate mesenchymal stem cells from other sources such as skeletal muscle, skin, or blood.

If desired, because MSCs are very rare cells (ranging for example from 0.01% to 0.001% of the total mononuclear cells of the bone marrow), said tissue sample may previously be depleted of the unrelated cells (for example haematopoietic and endothelial cells in the bone marrow) in order to further facilitate the detection and isolation of MSCs; this depletion can be performed by classical methods, using usual markers of the cells to be depleted (by way of non-limitative examples, markers such as CD 11b, CD 14, CD45, or CD235a can be used for depletion of hematopoietic stem cells).

According to a particular embodiment, the method of the invention comprises, in addition of the detection of the expression of CD200, the detection of the expression of at least one, preferably at least two, advantageously at least three, more preferably at least four, surface marker(s) selected among CD49b, CD105, CD130, CD146, and integrin alphaV/beta5. When used for isolating native mesenchymal stem cells from a tissue sample, the method of the invention further comprises isolating the cells which express this or these additional surface marker(s).

According to a particularly preferred embodiment, the method of the invention comprises the detection, and if needed, the isolation of cells that express CD200 and do not express CD105.

The detection of CD200, as well as the detection of the other surface markers that are mentioned herein, and the isolation of cells expressing said marker(s) can be effected by a number of different methods, which are known in themselves (for review cf. for instance HUNT, "Cell Separation Techniques Used in Immunology" In: ENCYCLOPEDIA OF LIFE SCIENCES. John Wiley & Sons, Ltd: Chichester, April 2001).

These methods generally rely upon binding of an antibody specifically recognizing the marker concerned. Cells that exhibit binding can then be identified and isolated.

Non-limitative examples of suitable technics include affinity chromatography, magnetic-activated cell sorting (MACS), "panning" with an antibody attached to a solid support, fluorescence-activated cell sorting (FACS), and combinations of two or more of these technics.

A further object of the invention is an isolated population enriched with CD200⁺ mesenchymal stem cells, obtainable by a method of the invention. The CFU-F frequency obtained from this population is at least 100, preferably at least 200, and still more preferably at least 300 fold times increased when compared to the CFU-F frequency obtained from the total bone marrow mononuclear cells.

The invention also encompasses an isolated CD200⁺ CD105⁻ native subset of mesenchymal stem cell, and an isolated CD200⁺ culture-amplified subset of mesenchymal stem cell.

The mesenchymal stem cell populations and isolated mesenchymal stem cells of the invention may further be genetically modified by introduction of exogenous genetic material, in particular by introduction of an exogenous gene of therapeutic interest. These genetically modified cell populations and cells are also part of the invention.

The invention also comprises therapeutic compositions comprising the mesenchymal stem cell populations or the isolated mesenchymal stem cells of the invention.

The mesenchymal stem cell populations, isolated mesenchymal stem cells, or therapeutic compositions of the invention may be used in all the cell-therapy applications of mesenchymal stem cells. They are particularly useful in the treatment of bone and cartilage defects or diseases (brittle bone disease, osteoarthritis...), the treatment of tendon rupture, the treatment of neurodegenerative diseases (Parkinson's disease, Hurler syndrome...), the treatment of bone marrow failure (primary or secondary to heavy radio/chemotherapy), the treatment of graft-versus-host disease after allogeneic hematopoietic stem cell transplantation, and of many other types of immunological diseases, the treatment of muscular diseases (myopathies, etc...), etc.

### LEGENDS OF THE DRAWINGS:

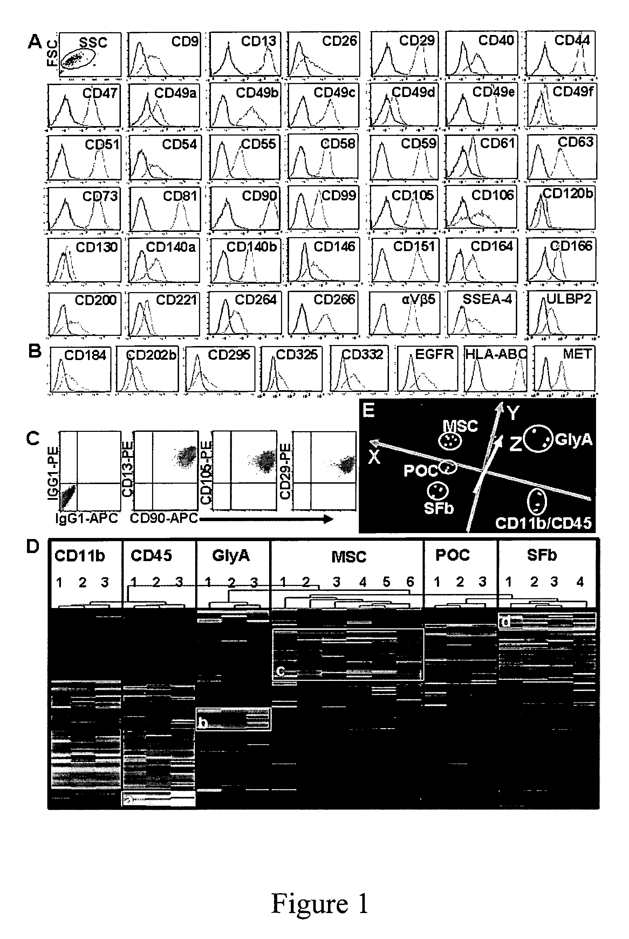

### Figure 1: Cultured BM MSCs

### A) Phenotype of P1 cells : positive markers detected using PE-conjugated mAbs.

Cells were collected at the end of passage 1 (day 42), after trypsinisation of confluent layers. Cells were incubated with phycoerythrin (PE)-conjugated mAbs added at saturating concentration, as indicated in Methods.

### B) Phenotype of P1 cells : positive markers detected using purified unconjugated mAbs.

For indirect staining, cells were incubated with the primary mouse anti-human mAbs, then with biotinylated goat anti-mouse Ig Abn and finally with R-Phycoerythrin (RPE)-conjugated streptavidin as indicated in Methods.

### C) Phenotype of P1 cells : single population by double labelling.

Cells prepared as indicated in Methods were labelled using APC-conjugated anti-CD90 and PE-conjugated anti-CD29 or anti-CD105 or anti-CD73.

### D) Hierarchical clustering.

Hierarchical clustering was performed using a panel of 147 CD transcripts (genes identified as "absent" by GCOS 1.2 in all samples were excluded), on 6 independent samples of P1 MSCs, 3 samples each of CD45+ (CD45), CD11b+ (CD 11b) and Glycophorin A+ (GlyA) hematopoietic cells, 3 samples of periosteal cells (POC), and 4 samples of synovial fibroblasts (SFb). Clusters : a: lymphoid cluster including CD2, CD3E, CD3Z, CD8A, CD117, CD122, CD132, CD160 and CD197 ; b : erythroid cluster including CD225a, CD234, CD240, CD241 and CD338 ; c : MSC cluster including CD49b, CD51, CD90, CD73, CD105, CD130, CD140a, CD140b, CD146, CD151, CD200, CD266, CD295, CD325 and CD332 ; d : SFb cluster including CD9, CD34, CD42b, CD62P, CD66d and CD227.

### E) Principal components analysis.

It was carried out using the same CD transcripts and on the same samples than for hierarchical clustering. Each plotted data point represents a single profile.

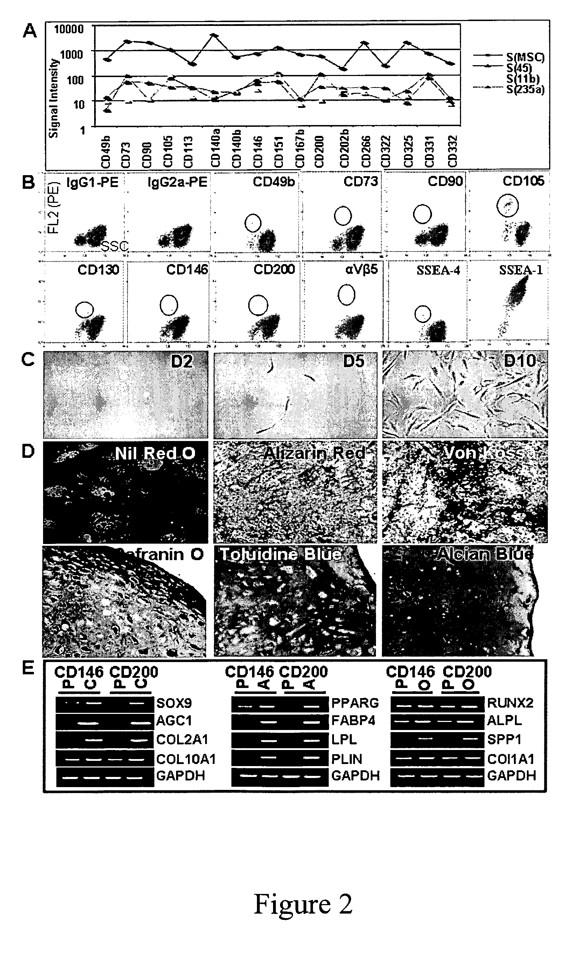

### Figure 2: Native BM MSCs

### A) FACS sorting.

BM mononuclear cells were prepared and labelled as indicated in Methods. The sorting gate was determined on the FL2 vs SSC as shown. The percentage of cells detected is indicated in each panel. For SSEA-1 no specific events were detected in the appropriate gate.

### B) CFU-Fs from CD200+ cells.

CD200⁺ sorted cells were cultured in alpha-MEM + 10% FCS + 1 ng/mL bFGF + supplements as indicated in Methods. Cultures were screened at day 2, 5 and 10. CFU-Fs were counted at day 10. CFU-Fs could not be grown from CD200-negative cells. Similar results were obtained for the different sortings using antibodies indicated in A.

### C) Adipocytic, osteoblastic and chondrocytic differentiation of CD200+ cells: histochemical markers.

P1 confluent layers obtained from CD200+ cells were trypsinized and cells were seeded in differentiation media as indicated in Methods. Adipocytic differentiation was assessed after 14 days by revealing the presence of cells containing large Nile Red O+ vesicles. Osteoblastic differentiation was assessed after 21 days by revealing the presence of von Kossa+ and Alizarin Red+ mineralized areas. Chondrocytic differentiation was assessed after 21 days by revealing the presence in the micropellets of cartilage-specific glycosaminoglycans stained by Safranin O, Toluidine Blue and Alcian Blue. Similar results were obtained for CD146+ cells.

### D) Adipocytic, osteoblastic and chondrocytic differentiation of CD200+ cells: molecular markers.

RNA was extracted from CD200+ cells cultured in proliferation medium (P) or differentiated into adipocytes (A), osteoblasts (O) and chondrocytes (C). Experiments were performed in parallel on CD200⁺ and CD146⁺ cells. RT-PCRs were performed using primers specific for :
C : transcription factor SOX-9 (SOX9), aggrecan core protein (AGC1), collagen 2, alpha1 chain (COL2A1), collagen 10, alpha1 chain (COL10A1),
A : peroxisome proliferator-activated receptor gamma (PPARG), fatty acid-binding protein (FABP4), lipoprotein lipase (LPL), perilipin (PLIN),
O: Runt-related transcription factor 2 (RUNX2), alkaline phosphatase (ALPL), osteopontin (SPP1), collagen 1, alpha1 chain (COL1A1).

Housekeeping gene: glyceraldehyde 3-phosphate dehydrogenase (GAPDH).

### Figure 3: Flow cytometry sorting of a native CD200⁺ CD105⁻ MSC sub-population:

BM mononuclear cells were prepared as indicated in Methods and co-labelled with CD200 PE-conjugated mAb and CD105 APC-conjugated mAb. A population of CD200⁺CD105⁻ cells, able to give rise to CFU-F, was identified and sorted.

### Figure 4: Flow cytometry sorting of a culture-amplified CD200⁺ MSC sub-population:

Culture-amplified cells were collected from confluent layers at the end of P 1 (day 36) and labelled with PE-conjugated CD200 mAB as indicated in methods. The PE-labelled cells were sorted using a MoFlo^{™} (Dako, 4850 Innovation drive, Fort Collins, CO, USA) high speed cell as indicated in methods. Sorting regions were defined on a dot-plot: fluorescence vs side scatter (SSC). Two populations of CD200+ and CD200- were identified and sorted.

### MATERIAL AND METHODS

### Cell culture

### Mesenchymal Stem Cells

BM cells were obtained from iliac crest aspirates from informed and consenting patients undergoing orthopedic surgery (Orthopedic Surgery department, Trousseau Hospital, Tours, France), following a procedure approved by the local ethical committee. Cells were centrifuged, nucleated cells were counted and seeded at a density of 5x10⁴ per cm² in alpha-MEM (Invitrogen, Cergy-Pontoise, France) supplemented with 10% (v/v) screened fetal calf serum (FCS), 1 ng/mL bFGF (AbCys), 20 µmol/L L-glutamine (Invitrogen) and 100 units/mL penicillin G (Invitrogen). Cells were incubated at 37°C in 95% humidified air and 5% CO₂. On day 3, all non-adherent cells were removed by changing the medium; thereafter, medium was changed twice a week. When fully confluent, the layer of adherent cells was trypsinized (0.25% v/v trypsin-EDTA, Invitrogen), the cells were resuspended in culture medium and seeded at 1x10³ cells per cm² (passage 1, PI). The three-lineage mesenchymal differentiation of the BM MSCs used was systematically checked by culturing cells in adipogenic, chondrogenic and osteogenic induction media as previously described (DELORME & CHARBORD : Culture and characterisation of human bone marrow mesenchymal stem cells. *Methods in Molecular Medicine, 2nd ed.: Tissue Engineering*. Edited by: H. Hauser and M. Fussenegger © Humana Press Inc., Totowa, NJ), and the lack of remaining hematopoietic CD45+ cells was verified by flow cytometry and molecular analyses.

### Synovial fibroblasts

Synovial fibroblasts were isolated from synovial tissues post-mortem (normal joints). Tissues were finely minced and digested with 0.2 % w/v collagenase in Dulbecco's minimum essential medium (DMEM) containing 10 % FBS, 100 U/ml penicillin and 100 µg/ml streptomycin (complete DMEM). Following 3-4 hours of incubation at 37°C, cells were collected by centrifugation, washed once and filtrated on nylon membrane (Cell Strainer; Dutscher, Cergy, France). Subsequently the cell suspension was plated in complete DMEM in a 75 cm² flask and passaged when reaching near confluence. Synovial cells were used at P4.

### Periosteal cells

Periosteal autografts (0.5 cm²) were harvested from human mastoid of three patients undergoing mastoidectomy. Periosteal tissues were rinsed with PBS (Biochrom, Berlin, Germany) and Hanks solution (Biochrom), minced, and subsequently digested in DMEM/Ham'sF12 medium (Biochrom) containing 10,000 U/ml collagenase II (Biochrom), 10% human allogenic serum (German Red Cross, Berlin) and 1% antibiotic-antimycotic solution (Biochrom). Tissue was digested for three hours at 37° C and cells were subsequently harvested, resuspended in DMEM/HamsF12 medium containing 10% human allogenic serum, plated, and allowed to attach for 4-10 days. Non-adherent cells were removed by exchange of medium. Adherent growing POCs were cultured under standard cell culture conditions and the medium was replaced every 2-3 days. When reaching about 90% confluence, POCs were sub-cultured by treatment with 0.5% trypsin-EDTA (Biochrom) and subsequently replated at a density of 6000 cells/cm². Culture homogeneity of POCs was verified by FACS analysis of relevant markers (CD44⁺, CD73⁺, CD105⁺, CD90⁺, CD14⁻, CD34⁻, CD45⁻). The progenitor character of these cells was demonstrated using standard assays routinely used for human BM MSCs (DELORME & CHARBORD, cited above).The study was approved by the ethical committee of the Charité-Unversitätsmedizin Berlin.

### In vitro differentiation

The differentiation potential of human BM MSCs was evaluated both by histochemical methods described in DELORME & CHARBORD (cited above) and by the analysis of differentiation-specific molecular markers.

RT-PCR reactions were performed using SuperScript One-Step RT-PCR with Platinum Taq (Invitrogen) according to the manufacturer instructions. The markers analyzed were :
- House-keeping gene:
   - glyceraldehyde 3-phosphate dehydrogenase (GAPDH)
- Osteogenic differentiation:
   - alkaline phosphatase (ALPL)
   - osteopontin (SPP1)
   - collagen type I, alpha1 chain (COL1A1)
   - runt-related transcription factor 2 (RUNX2)
- Adipogenic differentiation:
   - peroxisome proliferator-activated receptor-γ (PPARG)
   - fatty acid binding protein 4 (FABP4)
   - lipoprotein lipase (LPL)
   - perilipin (PLIN)
- Chondrogenic differentiation:
   - collagen type II, alpha1 chain (COL2A1)
   - collagen type X, alpha1 chain (COL10A1)
   - transcription factor SOX-9 (SOX9) BD
   - aggrecan core protein (AGC1)

All total cellular RNA samples were extracted with RNA easy kit (Qiagen SA., Courtaboeuf, France) from BM MSCs cultured in differentiation medium at the end of P1 (day 42). For differentiation-specific genes 50 ng of total RNA were used and for GAPDH gene 5 ng. RT-PCR products were analysed with agarose gel electrophoresis.

### Flow cytometry analysis

It was performed on either adherent, culture amplified cells or on fresh BM mononuclear cells. Culture-amplified cells were collected from confluent layers at the end of P1 (day 36) after incubation with 0.25% Trypsin-EDTA for 3 min. BM mononuclear cells were obtained after separation on Ficoll-Hypaque. Cells were washed once in PBS before staining.

For direct staining, cells were centrifuged (250 g, 5 min) and resupended in cold PBS. 10⁵ cells were incubated for 30 min at 4°C in the dark in 200 µl cold PBS with phycoerythrin (PE)-, allophycocyanin (APC), or fluorescein-conjugated monoclonal antibodies added at saturating concentration (see Suppl Table 7). Cells were then washed twice by centrifugation (250 g, 5 min) and resuspended with cold PBS, before proceeding with flow cytometric analysis. Acquisitions were performed on a FACSCalibur^{®} flow cytometer equipped with 488 nm argon laser. For indirect staining, cells were incubated at 4°C for 45 min with the primary mouse anti-human mAbs. Cells were subsequently incubated at 4°C with biotinylated goat anti-mouse Ig antibody (Becton-Dickinson) for 30 min and then with R-phycoerythrin (RPE)-conjugated streptavidin (Dako, Trappes, France) for 30 min. As negative control, cells were incubated with the corresponding irrelevant mouse IgG1, IgG2a, IgG2b or IgGM mAbs; a minimum of 20,000 events was recorded for each analysis, using a FACScalibur^{®} (Becton Dickinson) flow cytometer. Expression of membrane receptors was studied on a FL2-H logarithmic scale. For each antibody the histogram was compared to that of the irrelevant negative control. The mean fluorescence intensity (MFI) for the antigen of interest was related to that for the irrelevant isotypic control.

### Cell sorting

In order to determine transcripts specific for MSCs versus the other hematopoietic cell fractions (i.e: CD235a⁺ cells, CD45⁺ cells and CD11b⁺ cells), each fraction has been isolated from fresh human bone marrow samples using MACS (Miltenyi) according to the manufacturer's instructions. After a ficoll gradient, BM mononuclear cells were passaged successively through LS columns using magnetic beads conjugated to the respective antibodies against: 1) glycophorine A (CD235a), 2) CD45, and 3) CD11b). Cells retained by the columns were eluted and passaged a second time to ensure a maximal purity of each cell fraction. The final purity checked using flow cytometry with the corresponding antibodies was > 98%. Total cellular RNA of each samples were extracted with RNA easy kit (Qiagen SA., Courtaboeuf, France).

The remaining PE-labelled cells were sorted using a MoFlo^{™} (Dako, 4850 Innovation drive, Fort Collins, CO, USA) high speed cell sorter equipped with a solid state laser operating at 488 nm and 100 mW. We use morphological criteria to take off debris. R-phycoerythrin fluorescence was analysed with a 580/30-nm band pass filter. Sorting regions were defined on a dot-plot: fluorescence vs side scatter (SSC). Sorting speed was 10000 cells/sec. For the most accurate sorting we used the "purified mode" and a droplet envelope of one drop.

CFU-F assays were carried out on BM mononuclear cells and on cells sorted by FACS. Mononuclear cells were seeded in 25-cm² flasks at 3 different densities (2x10³, 4x10³ and 40x10³/cm²). Cultures were incubated for 10 days, the medium was then removed and cells were washed twice with PBS, fixed in methanol for 5 minutes and counterstained with Giemsa solution for 10 minutes. Colonies of 50 or more cells were counted. Sorted cells were seeded at a density ranging from 10²/cm² to 5x10²/cm².

### EXAMPLE 1: PATTERN OF MEMBRANE PROTEIN EXPRESSION IN CULTURED MSCS

We determined the transcripts encoding outer plasma membrane proteins (excluding Golgi, mitochondriae...) using Affymetrix microarrays in 6 P1 samples (day 42). Out of 1621 inventoried molecules, we detected 465 transcripts including 98 CD. Among the transcripts were 118 channel or transporter proteins, 102 cell-cell or cell-matrix adhesion receptors, 57 cytokine receptors and 20 junction molecules (tight, gap...). Most of the receptors specific for immune cells (T-cells, B-cells, NK-lymphocytes, dendritic cells and monocytes/macrophages), and receptors for chemokines, hormones, neuromediators and neuropeptides were not detected.

We studied 96 membrane proteins by flow cytometry; 51 were detected (Fig 1A), among which adhesion receptors (integrins, immunoglobulin superfamily members, tetraspanins), diverse receptor tyrosine kinases and HLA-ABC (but not HLA-DR). A single population was apparent on double fluorescence plots of CD90 vs CD 13, CD29 or CD105 (Fig 1B). We then investigated whether levels of mRNAs were correlated to those of proteins by plotting the relative mean fluorescence intensity (rMFI) given by flow cytometry studies vs the signals determined by microarrays. The highly significant (r² = 0,453, p < 0,0001, n=56) regression plot of the log(rMFI) vs array signals indicated a parabolic relationship.

### EXAMPLE 2: THE POPULATION OF CULTURED BM MSCs IS CLEARLY DISTINCT FROM THOSE OF HEMATOPOIETIC CELLS.

A panel of 147 CDs was selected based on the precondition that at least one hybridisation experiment of any of the different cell types revealed a "present" call. Hierarchical clustering indicated that cultured MSCs belonged to a cluster clearly distinct from those of hematopoietic cells (Fig 1C). Moreover, the population of cultured MSCs did not contain discernible cell subsets and was distinguishable from other skeletal, but non BM-derived, cultured mesenchymal cells, i.e. periosteal cells (POCs) and synovial fibroblasts (SFbs). The 3 first components of the principal component analysis (PCA) confirmed the distinctions (Fig 1D): hematopoietic cells were discriminated from mesenchymal cells according to the X-axis, while Y and Z-axes allowed to distinguish the different hematopoietic (CD45⁺, CD11b⁺ and CD235a⁺) and mesenchymal (MSC, POC and SFb) populations.

Out of 465 transcripts (including 98 CD) expressed in cultured MSCs, 113 (including 20 CD) were not or only at low level detected in hematopoietic cells. These were defined as specific for cultured MSCs. Of the 20 specific CD, 17 could be studied at the protein level and were expressed at the cell membrane (Fig 1A). The 15 molecules that discriminated cultured MSCs from hematopoietic cells were CD49b, CD90, CD73, CD105, CD130, CD140a, CD140b, CD146, CD151, CD200, CD266, CD295, CD325, CD332 and integrin alphaV(CD51)/beta5(ITGB5). Five of these (CD146, CD200, CD295, CD325, CD332) discriminated also, at least at the transcript level, MSCs from POCs and SFbs.

Our data demonstrate that cultured cells constitute a lineage-homogenous cell population. The mesenchymal lineage was clearly distinct from the hematopoietic lineages. 24,5 % (113/465) of the transcripts for membrane protein antigens were detected on cultured MSCs but not or only at low level on hematopoietic cells. Hierarchical clustering and PCA show that the different hematopoietic cell populations segregate apart from cultured MSCs and show no major gene expression variability from one MSC sample to the other. Clustering also discriminates MSCs from POCs and SFbs. Finally, MSCs present as a unique cell population that expresses the specific membrane antigens CD90 and CD105.

Lineage homogeneity does not preclude clonal heterogeneity since primary layers result from the expansion of CFU-Fs with highly variable proliferation and differentiation potential (BRUDER et al., J Cell Biochem, 64, 278-94, 1997; OWEN et al., J Cell Sci, 87 ( Pt 5), 731-8, 1987; PITTENGER et al., Science, 284, 143-7, 1999). Some of the clonogenic cells with high differentiation and proliferation potential and with self-maintenance capacity may be considered as bona fide stem cells: RS-1 cells in humans (COLTER et al., Proc Natl Acad Sci U S A, 98, 7841-5, 2001), SSEA-1+ cells in mice (ANJOS-AFONSO & BONNET, Blood, 109, 1298-306, 2007). Our work was not aimed at dissecting the different compartments of stem cells, transit amplifying progenitors, and mature cells. For cell therapy, it is essential to transplant a well-defined and homogenous cell population, as obtained in our study. Our standardized protocol, similar to that described by Pittenger et al. (PITTENGER et al., Science, 284, 143-7, 1999), has been upscaled to provide sufficient cells in high quality with known phenotype and differentiation potential for therapeutic administration.

### EXAMPLE 3: ANALYSIS OF MEMBRANE PROTEINS ON BONE MARROW MONONUCLEAR CELLS ALLOWS TO DEFINE THE CELL POPULATION OF ORIGIN

We hypothesized that some of the specific membrane antigens present on cultured MSCs would define the BM mesenchymal cell population of origin containing colony-forming units-fibroblasts (CFU-Fs). Of the 17 membrane proteins specific for cultured MSCs, 6 reproducibly allowed to determine a minute population of BM mononuclear cells (with low SSC and FSC) containing the CFU-Fs: CD49b, CD73, CD90, CD105, CD130, CD146, CD200 and integrin alphaV/beta5 (Fig 2A). The percentage (mean ± SEM, n=7) of BM cells recovered from the total MNCs varied from 1,9 ± 0,4% (CD105) to 0,15 ± 0,04% (CD200) indicating that some of the antibodies, such as CD105, selected a population encompassing, but not being restricted to, native MSCs

CD200 appeared to be one of the most distinctive marker for cultured MSCs and allowed to purify native MSCs reproducibly. Therefore, we studied the differentiation potential of CFU-Fs derived from CD200⁺ cells and found that the adipogenic, osteogenic and chondrogenic potential was similar to that observed for cells separated by adherence or according to CD146 expression (Fig 2B-D).

Four of the 5 surface markers for MSC that we identified - CD49b, CD105, CD146, integrin alphaV/beta5 - recognize *in vivo* vascular smooth muscle cells or pericytes. This suggests that native BM MSCs are cells associated to the vessel wall, a hypothesis previously advocated (BIANCO et al., Stem Cells, 19, 180-92, 2001; SHORT et al., Arch Med Res, 34, 565-71, 2003; DENNIS & CHARBORD, Stem Cells, 20, 205-14, 2002).

### EXAMPLE 4: USE OF CD200 FOR IDENTIFYING SUB-POPULATIONS OF NATIVE OR CULTURE-AMPLIFIED MSCs

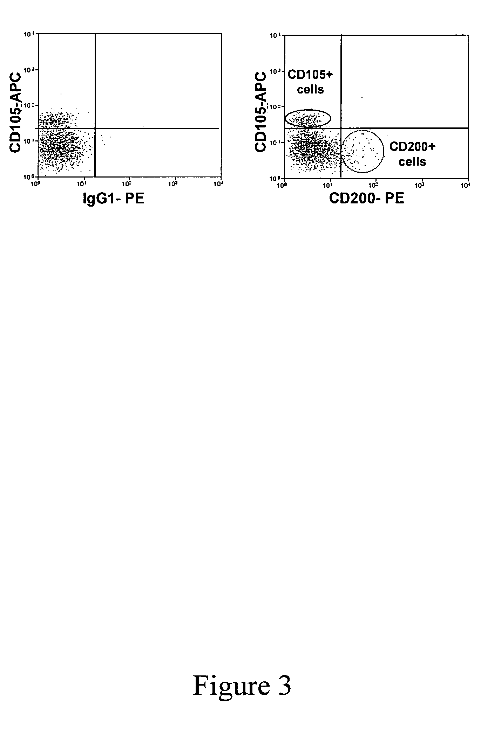

After depletion of bone marrow hematopoietic cell fractions (i.e: CD235a⁺ cells, CD45⁺ cells and CD11b⁺ cells), the remaining cells were co-labelled with CD200 PE-conjugated mAb and CD105 APC-conjugated mAb. A population of CD200+CD105- cells was identified and sorted using a using a MoFlo^{™} (Dako, 4850 Innovation drive, Fort Collins, CO, USA) high speed cell sorter (as describe in Material and Methods (see cell sorting)). CD200⁺CD105⁻ sorted cells were cultured in alpha-MEM + 10% FCS + 1 ng/mL bFGF + supplements as indicated in Methods. CFU-F were obtained from the CD200⁺CD105⁻ cells sorted by flow cytometry, indicating that CD200⁺ could identify a sub-population of MSC clearly distinct from the CD105⁺ cells. In addition, a very discrete double-stained (CD200⁺CD105⁺) MSC sub-population could also be identified. The results are shown in Figure 3.

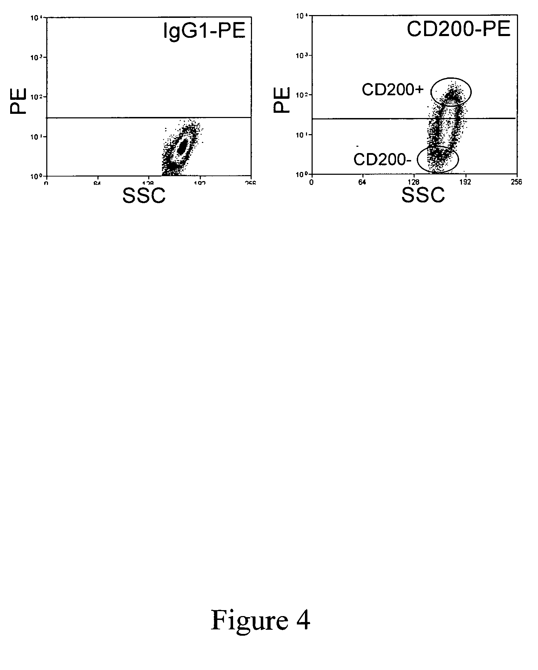

On culture-amplified MSCs, CD200 is also able to discriminate between the presence of CD200⁺ (high) and CD200- (or CD200low) cells, as shown in figure 4.

## Claims

1. A method for phenotyping a population of mesenchymal stem cells (MSCs), wherein said method comprises detecting the expression of the surface marker CD200 in this population.

2. A method for isolating native MSCs, wherein said method comprises providing a tissue sample containing MSCs, detecting the expression of CD200 in cells from this tissue sample, and isolating the CD200+ cells.

3. A method of claim 2 wherein said tissue sample is a bone marrow sample.

4. A method of any of claims 1 to 3, wherein said method comprises, in addition of the detection of the expression of CD200, the detection of the expression of at least one other surface marker selected among CD49b, CD73, CD90, CD105, CD130, CD 146, and integrin alphaV/beta5.

5. A method of any of claims 2 to 4, wherein said method comprises isolating the cells which express at least one surface marker selected among CD49b, CD73, CD90, CD105, CD130, CD146, and integrin alphaV/beta5.

6. A method of any of claims 1 to 4, wherein said method comprises the detection of cells that express CD200 and do not express CD105.

7. A method of claim 6, wherein said method comprises isolating the cells which express CD200 and do not express CD105.

8. An isolated population of MSCs obtainable by a method of any of claims 2 to 7.

9. An isolated population of claim 8, wherein said population is enriched with CD200⁺ CD105⁻ MSCs.

10. An isolated CD200⁺ CD105⁻ mesenchymal stem cell.

11. An isolated population of MSCs of any of claims 8 or 9, wherein said cells have been genetically modified by introduction of an exogenous oligonucleotide.

12. An isolated MSC of claim 10, which has been genetically modified by introduction of an exogenous oligonucleotide.

13. A therapeutic composition comprising an isolated population of MSCs of any of claims 8, 9 or 11, or an isolated MSC of any of claims 10 or 12.
